(19) Europäisches Patentamt
European Patent Office
Office européen des brevets

(11) EP 3 834 716 A1

(12) EUROPEAN PATENT APPLICATION

(43) Date of publication:
16.06.2021 Bulletin 2021/24

(51) Int Cl.:
A61B 5/053 (2021.01)    A61B 5/16 (2006.01)
A61B 5/00 (2006.01)

(21) Application number: 19215419.3

(22) Date of filing: 11.12.2019

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(71) Applicant: Koninklijke Philips N.V.
5656 AG Eindhoven (NL)

(72) Inventors:
• OUWERKERK, Martin
  5656 AE Eindhoven (NL)
• PENNING DE VRIES, Hendricus Theodorus
  Gerardus
  Maria
  5656 AE Eindhoven (NL)

(74) Representative: Philips Intellectual Property &
Standards
High Tech Campus 5
5656 AE Eindhoven (NL)

(54) WEARABLE SENSOR DEVICE AND A SENSING METHOD

(57) The present invention relates to a wearable sensor device and method for a thermogenic and/or psychogenic stress response of a subject. The wearable sensor device comprises a first electrode (11) configured to contact a first non-glabrous skin part of the subject, a second electrode (12) configured to contact a second non-glabrous skin part of the subject, a third electrode (13) configured to contact a glabrous skin part of the subject, and a skin conductance sensor (14) configured to provide a voltage signal between a positive input terminal (141) and a negative input terminal (142) and to measure a skin conductance signal at an output terminal (143). A switching arrangement (15) switches connections between the first to third electrodes and the positive and negative input terminals of the skin conductance sensor between at least three switching states. A processing unit (15) determines a thermogenic and/or psychogenic stress response from the measured skin conductance signals during the at least three switching states.

FIG.3

**Description**

FIELD OF THE INVENTION

The present invention relates to a wearable sensor device and a sensing method for determining a thermogenic and/or psychogenic stress response of a subject.

BACKGROUND OF THE INVENTION

There are two main causes for sweating: thermoregulation and emotional stress. The first type of sweating is called thermogenic sweating and the second type of sweating is called second psychogenic sweating. Thermogenic sweating is the result of the activation of the sweat glands by signals from the sympathetic autonomous nervous system. These signals are reported to occur at a frequency exceeding 1 Hz, which is too fast for individual skin conductance responses to show. As a result of thermogenic sweating the skin conductance level rises without discernible individual skin conductance responses, i.e. the skin conductance rises steeply with a lack of individual peaks when the person is sweating as a result of intense activity, such as climbing a set of stairs. Such sweating is caused by thermoregulation.
Using a wearable sensor device the activation of sweat glands can be measured by measuring the skin conductance. For lifestyle applications it is advantageous to know the responses of a person to emotional or physiological stressors. The skin conductance data contain this information, but may also contain the skin conductance variations caused by thermoregulation as described above. A known wearable sensor device, as e.g. described in M. Ouwerkerk, P. Dandine, D. Bolio, R. Kocielnik, J. Mercurio, H. Huijgen, J. Westerink, Wireless multi sensor bracelet with discreet feedback, Proceedings of Wireless Health 2013, Baltimore, USA, paper A6, measures the skin conductance at the volar side (also called underside) of the wrist.
Sweat gland activation can be the response of the body to entirely different stimuli. Heat or physical activity can raise the core body temperature, which can trigger a thermoregulation process that among other things activates sweat glands. Psychological or pain stimuli are other known triggers that activate sweat glands. From skin conductance measurements it is, however, not yet known how to quantify and separate the two contributions to sweat gland activity.

SUMMARY OF THE INVENTION

It is an object of the present invention to determine the effect of thermal and/or emotional stress of a person.
In a first aspect of the present invention a wearable sensor device for determining a thermogenic and/or psychogenic stress response of a subject is presented, the wearable sensor device comprising:

- a first electrode configured to contact a first non-glabrous skin part of the subject,
- a second electrode configured to contact a second non-glabrous skin part of the subject,
- a third electrode configured to contact a glabrous skin part of the subject,
- a skin conductance sensor configured to provide a voltage signal between a positive input terminal and a negative input terminal and to measure a skin conductance signal at an output terminal,
- a switching arrangement configured to switch connections between the first to third electrodes and the positive and negative input terminals of the skin conductance sensor between at least three switching states, wherein in each switching state two of the first to third electrodes are connected to the negative input terminal and the third of the first to third electrodes is connected to the positive input terminal or in each switching state two of the first to third electrodes are connected to the positive input terminal and the third of the first to third electrodes is connected to the negative input terminal, and
- a processing unit configured to determine a thermogenic and/or psychogenic stress response from the measured skin conductance signals during the at least three switching states.

In further aspects of the present invention, there are provided a corresponding sensing method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.
Preferred embodiments of the invention are defined in the dependent claims. It shall be understood that the claimed method, computer program and medium have similar and/or identical preferred embodiments as the claimed wearable sensor device, in particular as defined in the dependent claims and as disclosed herein.
The present invention is based on the idea to present a wearable sensor device and a sensing method that measure the effect of thermal and/or emotional stress of a person. This is done by semi- (or near-)simultaneous measurement of the skin conductance at a glabrous skin part and a non-glabrous skin part, e.g. at the upper side and the underside of the wrist. Skin conductance is a means to monitor sweat gland activation by the sympathetic branch of the autonomic nervous system, which becomes active as a result of stress. Analogous to sweating behavior at both sides of a hand, thermal stress activates the majority of sweat glands at the upper side of the wrist (or another non-glabrous skin part) and emotional stress at the underside of the wrist (or another glabrous skin part). These semi-simultaneous measurements are subject to crosstalk but with the proposed commutation method, it is possible to eliminate this crosstalk. Through the switching of the connections between the electrodes and the skin conductance sensor the polarity of the electrodes is switched according to a fixed pattern such that average electrode current flow approaches zero.

The measurements are semi-simultaneous as they are not taken at the same moment in time but follow each other after short delay. The delay between measurements should be short and in relation with the bandwidth of the signals that are measured that is significantly shorter than the rate of signal changes of the conductance signals.

Using an appropriate transformation the conductance or resistance of each electrode-skin contact can be calculated, from which the contributions of thermogenic sweating and psychogenic sweating to the skin conductance signals can be derived.

Generally, there is no single conductance value applicable to a subject, but the measured conductance depends on the subject itself as well on the actual measurement position and the subject's stress and activity levels. Thus, measuring semi-simultaneous at different positions, as provided according to the present invention, allows separating between conductance contributions from stress and activity sources.

A raise in cortisol caused by psychological stress may be calculated using a mental balance method described in US 10085695. It has been found by the inventors that the cortisol response to physical activity is significantly smaller than the cortisol response to psychological stimuli. In order to accurately implement the known mental balance method both the thermogenic and the psychogenic contributions to sweat gland activation should be quantified. The present invention enables this and can thus be used in the known mental balance method to more accurately determine the cortisol response to stressors.

In an embodiment the switching arrangement is configured to switch connections between the first to third electrodes and the positive and negative input terminals of the skin conductance sensor between six switching states, wherein in each of three switching states two of the first to third electrodes are connected to the negative input terminal and the third of the first to third electrodes is connected to the positive input terminal and in each the other three switching states two of the first to third electrodes are connected to the positive input terminal and the third of the first to third electrodes is connected to the negative input terminal.

If an ideal offset free conductance sensor is used and the skin conductance of the subject is modeled as a pure passive electrical network, almost identical results will be expected from the measurements in the first three switching states and in the second three switching states. However, with small conductance sensor offsets differences in these results are expected which may be useful in order to suppress offset effects and improve the measurement accuracy. Further, with measurements from the six switching states a more complex skin conductance model that e.g. includes voltage sources may be solved. Generally, with N independent measurements N model parameters can be solved. The exact formula to apply the compensations depends on the skin conductance sensor electronics design.

In another embodiment the switching arrangement is configured to switch connections between the first to third electrodes and the positive and negative input terminals of the skin conductance sensor, after the at least three switching states, into a DC-free switching state, in which the first to third electrodes are all connected to the negative input terminal or the positive input terminal. Further, for DC-free operation all switching states not having all electrodes shorted should have equal duration. In this way, a DC-free measurement can be performed and it can be prevented that DC current is flowing into the measurement subject. In addition, a DC-free operation (on average) may further be guaranteed if each measurement cycle has the same duration. A simplest measurement cycle repeats the measurements in the above-described three or six switching states. In particular implementations, it may be desired to have repeating cycles of four measurements so that after the three switching states a DC-free switching state is used that do not disturb the DC-free behavior.

The switching arrangement may comprise three switches, each having a first terminal connected to a respective one of the first to third electrodes and two second terminals connected to the positive input terminal and the negative input terminal of the skin conductance sensor. In another embodiment that provides more flexibility, the switching arrangement may comprise six switches, wherein three switches each have a first terminal connected to a respective one of the first to third electrodes and a second terminal connected to the positive input terminal of the skin conductance sensor and the other three switches each have a first terminal connected to a respective one of the first to third electrodes and a second terminal connected to the negative input terminal of the skin conductance sensor.

In an embodiment the processing unit is configured to determine the thermogenic stress response and/or psychogenic stress responses by evaluating heights of rising edges in the measured skin conductance signals. In particular, the processing unit may be configured to determine that an increase in skin conductance results from thermogenic stress if heights of rising edges are higher in a skin conductance signal obtained from a non-glabrous skin region than in a skin conductance signal obtained from a glabrous skin region and/or to determine that an increase in skin conductance results from psychogenic stress if heights of rising edges are higher in a skin conductance signal obtained from a glabrous skin region than in a skin conductance signal obtained from a non-glabrous skin region.

Thus, if heights of rising edges (simultaneously appearing in different skin conductance signal measured from a glabrous skin region and a non-glabrous skin region) are higher in a skin conductance signal obtained from a non-glabrous skin region, these heights are considered as thermogenic stress response, i.e. as resulting from thermal stress. The switching frequency should thus be fast compared to the rate of change in the skin conductance signal. If the switching speed is too slow it may become impossible to detect simultaneous rising edges in the various switching state signals. If heights of rising edges (simultaneously appearing in different skin conductance signal measured from a glabrous skin region and a non-glabrous skin region) are higher in a skin conductance signal obtained from a glabrous skin region, these heights are considered as psychogenic stress response, i.e. as resulting from emotional stress.

Hence, the determination of the thermal stress level is done using solely these rising edges. The thermal stress level may be calculated by identifying skin conductance peaks and determining a psychogenic stress level of the user based on normalized parameters of the identified skin conductance peaks. The emotional stress level can be determined similar as the thermal stress level.

The cumulative stress load can be linked to the contribution to the cortisol level. A method to calculate it from rising edges heights is e.g. described in US 10085695, according to which a stimulus response is determined in a skin conductance signal and an estimated cortisol level trace is determined from the stimulus response. The estimated level of mental balance or imbalance is then determined from the estimated cortisol level trace. The ratio of cortisol generation linked to thermal rising edges and cortisol generation linked to emotional rising edges can be quantified. As exemplary ratio the value of 1/3.6 may be used.

In another embodiment the switching arrangement may be configured to switch the connections at a switching rate that is an integer fraction of the sampling rate of the measured skin conductance signal.

Depending on signal quality, it may not be needed to implement full time conductance measurement. Measurement currents can be reduced by duty-cycling the individual measurements. In such embodiment, each individual measurement period can be followed by an 'all electrodes shorted' cycle. The 'active' period of the conductance measurement cycles, not having all electrodes shorted, should be kept equal in order to keep DC-free operation.

In one embodiment the first, second and third electrodes are integrated into a single device. Hence, the electrodes can all be mounted to the subject's body at once.

In another embodiment the first, second and third electrodes are configured to be arranged separately at the subject's body. This provides more flexibility of mounting the electrodes to desired positions at the subject's body. Preferably, the processing unit may then be configured to synchronize evaluation of the skin conductance signals measured in the different switching states.

In an advantageous implementation the wearable sensor device further comprises a strap configured to be wound around the subject's wrist and a housing held by the strap and housing the switching arrangement, the skin conductance sensor and the processing unit, wherein the first and second electrodes are arranged in or at a bottom surface of the housing facing the dorsal side (also called upper side) of the wrist when the wearable sensor device is worn by the subject, and wherein the third electrode is arranged in or at a position of the strap facing the volar side of the wrist when the wearable sensor device is worn by the subject. The wearable sensor device may thus be configured like a wrist-worn watch or fitness device.

In another embodiment the switching arrangement may be configured to apply switching states, which individually or on average have an equal duration. For instance, the (long term average) duration of active switch states (e.g. states not having all electrodes shorted) should be equal in order to enable DC-free operation.

In one or more further embodiments a temperature sensor and/or an accelerometer may be provided for arrangement to the subject's body to get further information. A temperature sensor can offer additional information pertaining to the occurrence of thermoregulation. A 3D accelerometer can offer additional information pertaining to physical activity. Such embodiments may thus use universal knowledge that activity or sitting in the sauna can cause sweating to make sure that in such situations measurements are not misinterpreted or measurement are even avoided. Further, the disclosed deconvolution method should work irrespective of know-how of activity or ambience. The additional information offers insight in the person's thermoregulation threshold. It is known that the thermoregulation threshold shifts when moving from a cold to a hot climate for a prolonged period and vice versa. Hence, the extent of acclimatization can be monitored and taken into account in the evaluation of the skin conductance signal.

Further, a user interface (e.g. a display, a communication unit, etc.) may be provided to convey the thermogenic and/or the psychogenic stress response to the end user or to an application or device that utilizes the stress response information.

BRIEF DESCRIPTION OF THE DRAWINGS

These and other aspects of the invention will be apparent from and elucidated with reference to the embodiment(s) described hereinafter. In the following drawings

Fig. 1 shows a diagram illustrating measurement of skin conductance using two electrodes at the dorsal side of the wrist;
Fig. 2 shows a skin conductance signal to illustrate thermogenic and psychogenic contributions;
Fig. 3 shows a schematic diagram of a first embodiment of a wearable sensor device according to the present invention;
Fig. 4 shows a diagram of a second embodiment of a wearable sensor device according to the present invention in the form of a wrist-worn device;
Fig. 5 shows a diagram of a first embodiment of switching arrangement as used in a wearable sensor device according to the present invention;
Fig. 6 shows a schematic diagram of a cross-section of the wrist indicating various resistances;
Fig. 7 shows a diagram of six skin conductance signals measured in six different switching states;
Fig. 8 shows a diagram of a second embodiment of switching arrangement as used in a wearable sensor device according to the present invention;
Fig. 9A shows a diagram of psychogenic and thermogenic volar activation of sweat glands;
Fig. 9B shows a diagram of psychogenic and thermogenic dorsal activation of sweat glands;
Fig. 10 shows a diagram of a skin conductance signals measured at the dorsal side of the wrist and at the volar side of the wrist and of a composite signal of the two skin conductance signals;
Fig. 11 shows a flow chart of an embodiment of a method according to the present invention;
Fig. 12 shows a diagram of a skin conductance peak or skin conductance response;
Fig. 13 shows diagrams of a raw skin conductance trace signal, a filtered skin conductance trace signal and of skin conductance peaks identified therein;
Fig. 14 shows diagrams of a skin conductance trace signal, absolute rising edge heights and normalized rising edge heights extracts from said skin conductance signal trace;
Fig. 15 shows further diagrams of a skin conductance trace signal, absolute rising edge heights and normalized rising edge heights extracts from said skin conductance signal trace;
Fig. 16 shows diagrams of a skin conductance trace signal, determined stress level based on absolute rising edge heights and determined stress level based on normalized rising edge heights extracts from said skin conductance signal trace;
Fig. 17 shows a diagram of a skin conductance trace signal and a positive first order derivative or steepness of the absolute rising edge heights; and
Fig. 18 shows a diagram of a skin conductance trace signal and a positive first order derivative of a logarithmic transformed skin conductance.

DETAILED DESCRIPTION OF THE EMBODIMENTS

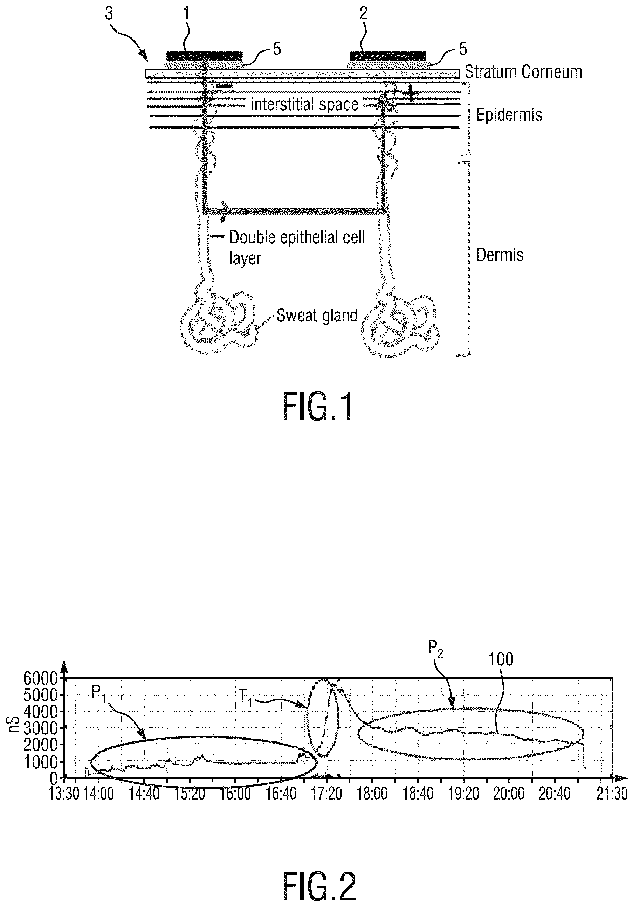

Fig. 1 shows a diagram illustrating measurement of skin conductance using two electrodes 1, 2 arranged to contact the skin 3, e.g. the volar side or the dorsal side of the wrist. The conductive path 4 between the electrodes 1, 2 through the skin is shown as well. A sweat layer 5 may be formed between the electrodes 1, 2 and the skin 3.

The ratio of thermogenic and psychogenic sweating can be determined for hairy (non-glabrous) and non-hairy (glabrous) skin, e.g. at the volar (palmar) and dorsal (upper) side of the hand. However, a better ratio is required: thermogenic sweating dominates psychogenic sweating on hairy parts of the skin on the outside of the wrist where the wearable sensor device may be worn. This effect is illustrated in Fig. 2 showing a typical skin conductance signal 100 (measured with a device having two electrodes contacting the dorsal side of the wrist) to illustrate thermogenic contribution $T_1$ and psychogenic contributions $P_1$, P2. As shown there, thermogenic sweating caused by activity dominates the skin conductance measurement.

In more detail, the first period $P_1$ (first period of psychogenic sweating) from about 13:50h to 17:10h denotes a time of psychogenic sweating, wherein the user was in a temperature-controlled environment and concentrated on a mental task but non engaging in physical activity. Sweating during this period $P_1$ can be attributed to psychogenic sweating. The second period $T_1$ (first period of thermogenic sweating) from about 17:10h to 17:30h denotes a period by thermogenic sweating, wherein the user engages in heavy physical activity (cycling for catching the train at 17:27h). Sweating during this period $P_2$ can be attributed to psychogenic sweating. The third period of time $P_2$ (second period of psychogenic sweating) again denotes a time in a temperature controlled environment without physical activity. Skin conductance responses in this time period may again be attributed to psychogenic sweating.

For determining a psychogenic stress level of a user, a skin conductance signal may be processed by identifying a plurality of skin conductance peaks in the skin conductance signal; determining, for each of said skin conductance peaks, a normalized parameter of said skin conductance peak, normalized based on a skin conductance value (i.e. a base level) of the respective skin conductance peak; and determining a psychogenic stress level of the user based on said normalized parameters of said skin conductance peaks. This method is impervious to skin conductance level changes such as those caused by physical activity or climate induced thermogenic sweating. Because of it being impervious the normalization prior to comparing rising edge heights may reduce or even nullify the differences between glabrous and non-glabrous locations. The present invention may be built on this concept and provides the ability to quantify and separate the two contributions to sweat gland activity.

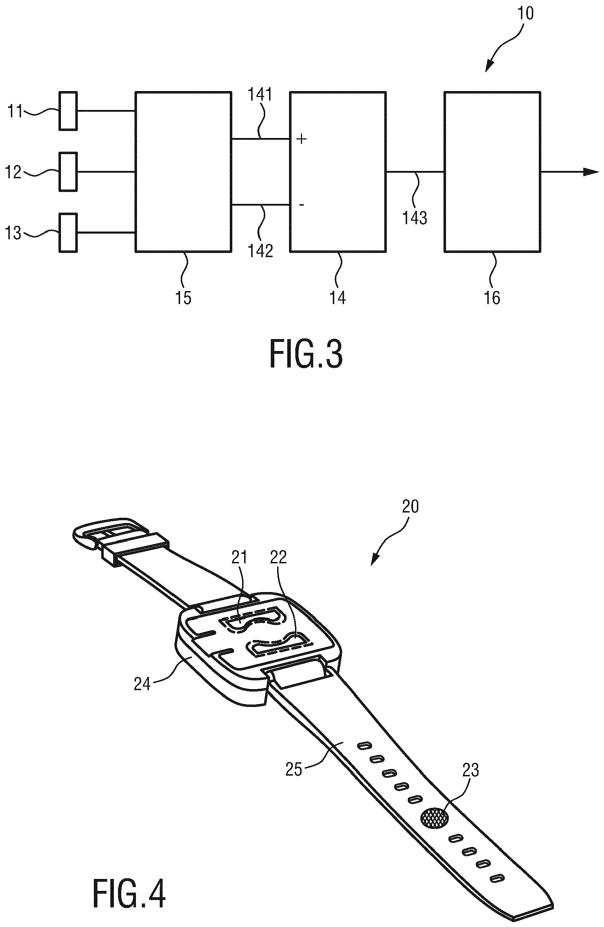

Fig. 3 shows a schematic diagram of a first embodiment of a wearable sensor device 10 according to the present invention. It comprises a first electrode 11 configured to contact a first non-glabrous skin part of the subject, a second electrode 12 configured to contact a second non-glabrous skin part of the subject, and a third electrode 13 configured to contact a glabrous skin part of the subject. The three electrodes may hereby be integrated into a common device. Alternatively, they may be separate elements that can separately be mounted at the subject's body

A skin conductance sensor 14 provides a voltage signal V between a positive input terminal 141 and a negative input terminal 142 and measure a skin conductance signal at an output terminal 143. Such a skin conductance sensor is generally known. It may comprise a voltage generator for applying the voltage between the terminals 141, 142, a sensing unit for sensing a current between the terminals, and/or a calculating unit for calculating the skin conductance based on the sensed current. The measured skin conductance over time forms the skin conductance time signal (also called skin conductance trace). The skin conductance signal (or data) may for example be stored in a memory of the wearable sensor device 10, or may be transmitted (wirelessly or through a wire or signal line) to an external unit.

A switching arrangement 15 is provided that switches connections between the first to third electrodes 11, 12, 13 and the positive and negative input terminals 141, 142 of the skin conductance sensor 14 between at least three switching states. In each switching state two of the first to third electrodes 11, 12, 13 are connected to the negative input terminal 142 and the third of the first to third electrodes 11, 12, 13 is connected to the positive input terminal 141. Alternatively, in each switching state two of the first to third electrodes 11, 12, 13 are connected to the positive input terminal 141 and the third of the first to third electrodes 11, 12, 13 is connected to the negative input terminal 141. Thus, the polarity of the current through the electrodes is regularly reversed. The switching arrangement may be implemented by analog or digital switching circuitry. Embodiments will be explained below.

A processing unit 16 determines a thermogenic and/or psychogenic stress response from the measured skin conductance signals during the at least three switching states. Details of an embodiment will be explained below. The processing unit may be implemented in hard- and/or software, e.g. by a computer, processor or an application program running on a computer or processor.

Fig. 4 shows a diagram of a second embodiment of a wearable sensor device 20 according to the present invention in the form of a wrist-worn device. The wearable skin conductance sensor 20 is fitted with two skin contact electrodes 21, 22 (representing the first and second electrodes) arranged at the surface of a housing 24 (or casing) of the device 20 so that they face the dorsal (upper) side of the wrist. Optionally a circular area may be provided in between the electrodes 21, 22 that can e.g. be used for the placement of another sensor, such as a photoplethysmograph heart rate sensor. An additional skin contact electrode 23 (representing the third electrode 13) is built into the strap 25 at a location that enables contacting the skin at the volar side (under side) of the wrist.

Wrist circumferences are known to differ substantially between persons so that it is vital to provide a means to either shift this additional skin contact electrode 23 to the proper (middle of the underside) position or to variably attach the strap to the housing 24, e.g. at two sides.

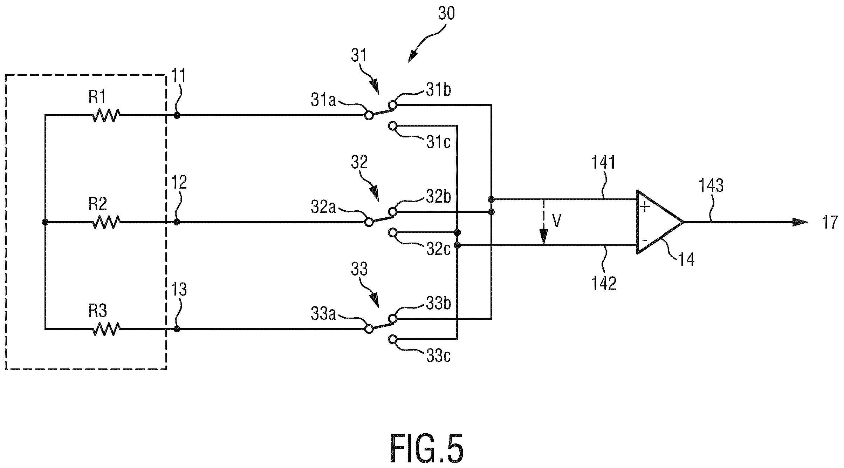

The skin conductance is measured by the wearable sensor device using two of the three electrodes 11-13 (Fig. 3) or 21-23 (Fig. 4) shorted. The third electrode 13 and 23 may be at 0.5 V (positive or negative) with respect to the other two electrodes 11, 12 and 21, 22, respectively. Fig. 5 shows a diagram of a first embodiment of switching arrangement 30, which may be used as switching arrangement 15 (as shown in Fig. 3) in a wearable sensor device according to the present invention. In this embodiment the switching arrangement 30 comprises three switches 31, 32, 33, each having a first terminal 31a, 32a, 33a connected to a respective one of the first to third electrodes 11, 12, 13 and two second terminals 31b, 31c, 32b, 32c, 33b, 33c connected to the positive input terminal 141 and the negative input terminal 142 of the skin conductance sensor 14 between which the voltage V is applied. The resistances of the part of the body to which the electrodes are mounted (e.g. the wrist) are indicated as resistances R1, R2, R3.

SPDT (single pole double throw) switches may be used for the switches 31, 32, 33. The switches 31, 32, 33 may be independently controlled, e.g. by a by a micro controller or the processor 16 (not shown in Fig. 5). The switching time is preferably synchronized with the sampling of the skin conductance signal 17.

In an embodiment a fourth switching state, in which all electrodes are shorted, may additionally be used. Table 1 shows eight switching states A-H of the three electrodes 11-13.

Table 1

| Switching state | Electrode 11 | Electrode 12 | Electrode 13 |
|---|---|---|---|
| **A** | + | - | - |
| **B** | - | + | - |
| **C** | - | - | + |
| **D** | - | + | + |
| **E** | + | - | + |
| **F** | + | + | - |
| **G** | + | + | + |
| **H** | - | - | - |

In an implementation, it can be distinguished between commutator switching rate, sample rate of individual conductance measurements Ra, Rb and Rc and report rate of the three biophysical conductance values R1, R2 and R3. In general, it is optimal to have commutation switching rate equal to conductance sampling rate. To estimate one or more biophysical conductance values, at least three individual conductance measurements should be made. In an implementation, the sampling rate of R1, R2 and R3 becomes effectively approximately one third of the sampling rate of Ra, Rb and Rc. Hence, the switching frequency should be fast compared to the rate of change in the skin conductance signal. If the switching speed is too slow it may become impossible to detect simultaneous rising edges in the various switching state signals. In case an additional dummy state is inserted into the commutation sequence the effective sampling rate of R1, R2 and -R3 becomes approximately one fourth of that of Ra, Rb and Rc.

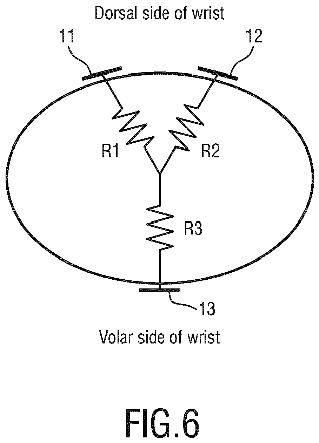

Fig. 6 shows a schematic diagram of a cross-section of the wrist indicating the various resistances R1, R2 and R3. The three electrodes 11, 12, 13 all contact the skin of the wrist. The skin conductances measured during the various switching states are related to the three skin resistances R1, R2 and R3. The interior of the wrist can, with respect to these resistances, be considered as a highly conductive medium, which is known to a person skilled in the art of biometry. Thus, the three skin resistances R1, R2 and R3 can be considered to be linked in the middle of the wrist as shown in Fig. 6. In the situation of the present invention two of the electrodes are also connected to each other on the dorsal side of the wrist and can thus be considered to be parallel resistors.

The resistances measured in the first three switching states A-C of Table 1 are linked to the three skin resistances as follows:

$$\text{Switching state A:} \quad Ra = R1 + (R2 \parallel R3)$$

$$\text{Switching state B:} \quad Rb = R2 + (R3 \parallel R1)$$

$$\text{Switching state C:} \quad Rc = R3 + (R1 \parallel R2)$$

For completeness the switching state D-F have similar relationships:

$$\text{Switching state D:} \quad Rd = R1 + (R2 \parallel R3)$$

$$\text{Switching state E:} \quad Re = R2 + (R3 \parallel R1)$$

$$\text{Switching state F:} \quad Rf = R3 + (R1 \parallel R2)$$

Using a transformation the skin resistances R1, R2 and R3 can be calculated from Ra, Rb and Rc as follows:

$$R1 = -(2 * Ra * Rb * Rc * (Ra + Rb + Ra * Rc - Rb * Rc))/(Ra^2 * Rb^2 - 2 * Ra^2 * Rb \\ * Rc + Ra^2 * Rc^2 - 2Ra * Rb^2 * Rc - 2 * Ra * Rb * Rc^2 + Rb^2 * Rc^2)$$

$$R2 = -(2 * Rb * Rc * Ra * (Rb * Rc + Rb + Ra - Rc * Ra))/(Rb^2 * Rc^2 - 2 * Rb^2 * Rc \\ * Ra + Rb^2 * Ra^2 - 2Rb * Rc^2 * Ra - 2 * Rb * Rc * Ra^2 + Rc^2 * Ra^2)$$

$$R3 = -(2 * Rc * Ra * Rb * (Rc + Ra + Rc * Rb - Ra * Rb))/(Rc^2 * Ra^2 - 2 * Rc^2 * Ra \\ * Rb + Rc^2 * Rb^2 - 2Rc * Ra^2 * Rb - 2 * Rc * Ra * Rb^2 + Ra^2 * Rb^2)$$

$$R1 \\ = \frac{2 * Ra * Rb * Rc * (Rb * Rc - Ra + Rb - Ra * Rc)}{Ra^2 * Rb^2 - 2 * Ra^2 * Rb * Rc + Ra^2 * Rc^2 - 2Ra * Rb^2 * Rc - 2 * Ra * Rb * Rc^2 + Rb^2 * Rc^2}$$

$$R2 \\ = \frac{2 * Rb * Rc * Ra * (Rc * Ra - Rb * Rc - Rb * Ra)}{Rb^2 * Rc^2 - 2 * Rb^2 * Rc * Ra + Rb^2 * Ra^2 - 2Rb * Rc^2 * Ra - 2 * Rb * Rc * Ra^2 + Rc^2 * Ra^2}$$

$$R3 \\ = \frac{2 * Rc * Ra * Rb * (Ra * Rb - Rc + Ra - Rc * Rb)}{Rc^2 * Ra^2 - 2 * Rc^2 * Ra * Rb + Rc^2 * Rb^2 - 2Rc * Ra^2 * Rb - 2 * Rc * Ra * Rb^2 + Ra^2 * Rb^2}$$

where R1, R2, R3 represent skin resistances from the electrodes to the electrical center of wrist and Ra, Rb, Rc represent the measured resistances between electrode 11 and shorted electrodes 12 and 13 (Ra), electrode 12 and shorted electrodes 13 and 11 (Rb) and electrode 13 and shorted electrodes 11 and 12 (Rc).

Alternatively, similar formula can be written for the conductances:

$$G1 = \frac{2 * Ga * Gb + 2 * Gb * Gc + 2 * Gc * Ga - Ga^2 - Gb^2 - Gc^2}{2 * (-Ga + Gb + Gc)}$$

$$G2 = \frac{2 * Gb * Gc + 2 * Gc * Ga + 2 * Ga * Gb - Gb^2 - Gc^2 - Ga^2}{2 * (-Gb + Gc + Ga)}$$

$$G3 = \frac{2 * Gc * Ga + 2 * Ga * Gb + 2 * Gb * Gc - Gc^2 - Ga^2 - Gb^2}{2 * (-Gc + Ga + Gb)}$$

where G1=1/R1, G2=1/R2 and G3=1/R3 and Ga=1/Ra, Gb=1/Rb and Gc=1/Rc.

Toggling between switching states A, B and C, each switching state with the same 'active' period, results in a long term average zero current through all three electrodes. The same is true for switching states D, E and F. As resistors are insensitive to voltage polarity, the same values will be measured for Ra, Rb and Rc as for Rd, Re and Rf. Mixing up states from both groups is allowed, but for average zero current both groups shall be fully used. Incomplete usage of one or both groups may lead to non-zero average current through the electrode pins.

Switching states G and H are not usable to measure skin conductance values. They short all electrodes together resulting in a zero current flowing through the electrodes. these switching states are still useful as a 'no operation' to allow for any length switching sequence.

Example sequences of switching states are:

OK:          repeat {A B C A B C F D E C A B}
OK:          repeat {A B C G}

(continued)

| OK: | repeat {A G B G C H} |
| OK: | repeat {D E F} |
| Undesired: | repeat {A B C A B C F D E C E B} |
| Undesired: | repeat {A B C C} |

The switching arrangement shown in Fig. 5 shows a simple electric circuit diagram with three resistors from the outer wrist to the wrist center. This circuit model ignores any voltage sources that may appear in real life situations due to static charge (hairs, movements), electro chemical activity, and/or voltages induced by nerve activities. These voltage sources may be added to the circuit diagram of Fig. 5. In that case every resistor R1, R2 and R3 gets a series voltage source E1, E2 and E3. These six unknowns (three voltages and three resistances) can be resolved uniquely if six switching states A-F are used and the appropriate transformation is applied with the six measured resistance values Ra, Rb, Rc, Rd, Re and Rf as input.

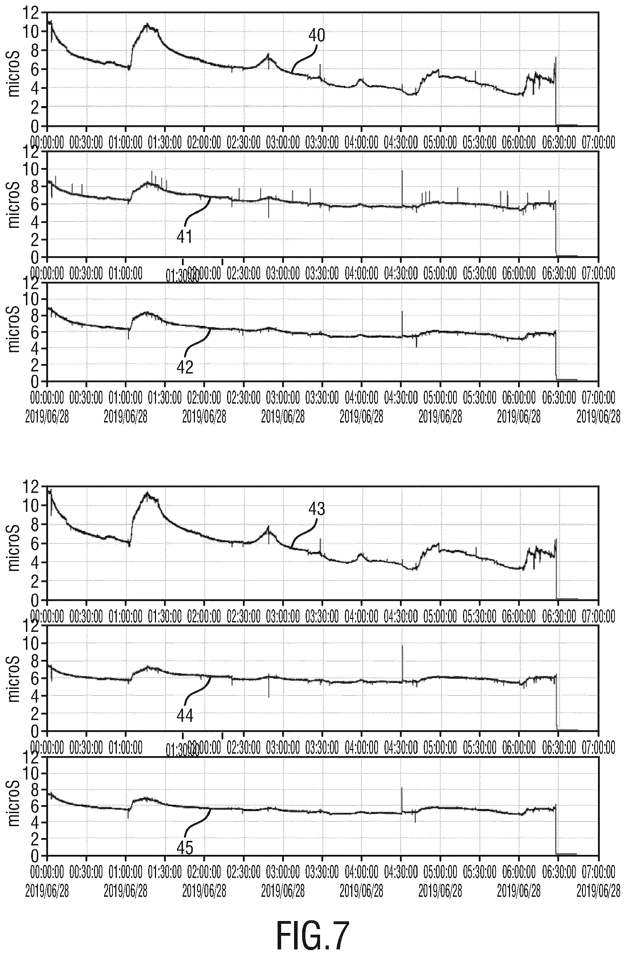

Fig. 7 shows a diagram of six skin conductance signals 40-45 measured in six different switching states A-F that are used in sequence. A comparison of the three switching states --+, -+- and +-- with their inverse states ++-, +-+ and -++ shows a consistent small (e.g. approx. 10%) difference in amplitude. These can be attributed to static charge (hairs, movements), electro chemical activity, and/or voltages induced by nerve activities.

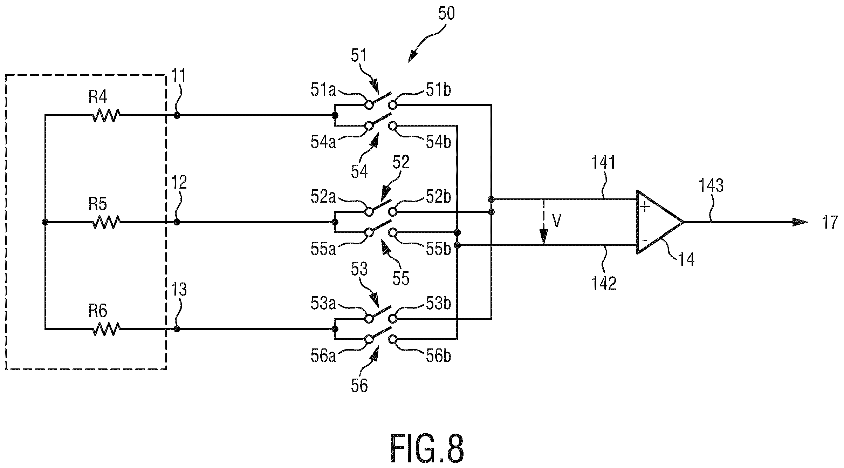

Two shortened electrodes are used according to the present invention to guarantee average zero current through the skin at shortest switching sequence. Fig. 8 shows a diagram of a second embodiment of switching arrangement 50, which may be used as switching arrangement 15 (as shown in Fig. 3) in a wearable sensor device according to the present invention. the switching arrangement 50 comprises six switches 51-56, wherein three switches 51-53 each have a first terminal 51a-53a connected to a respective one of the first to third electrodes 11-13 and a second terminal 51b-53b connected to the positive input terminal 141 of the skin conductance sensor 14 and the other three switches 54-56 each have a first terminal 54a-56a connected to a respective one of the first to third electrodes 11-13 and a second terminal 54b-56b connected to the negative input terminal 142 of the skin conductance sensor 14.

The switching arrangement 50 allows for 64 different switch positions from which 14 turn out to be meaningful. These 14 switching states mimic states A-H plus six additional switching states that mimic conventional DC based skin conductance measurement or commutation measurements between the electrode pairs. With switches 51 and 55 closed Rx = R4 + R5 is measured. With switches 51 and 56 closed Ry = R4 + R6 is measured. With switches 52 and 56 closed Rz = R5 + R6 is measured.

In this case the 'single ended skin resistances' can be estimated by another transform:

$$R4 = ½ * (Rx - Ry + Rz)$$

Mathematics are simple but there is no control over the average current flowing through the electrodes. Without shorted electrodes, zero average current can be guaranteed by traditional commutation or by emulation of the states in Table 1. For instance, after each measurement with switches 51 and 55 closed, a second measurement is preferred with reversed current that can be realized with switches 52 and 54 closed.

For a 3-electrode measurement a sequence of six switching states is used to guarantee zero current. In case of using shortened electrodes, the length of the switching sequence can be reduced to three switching states. This results in a higher sampling rates of the skin conductance signals.

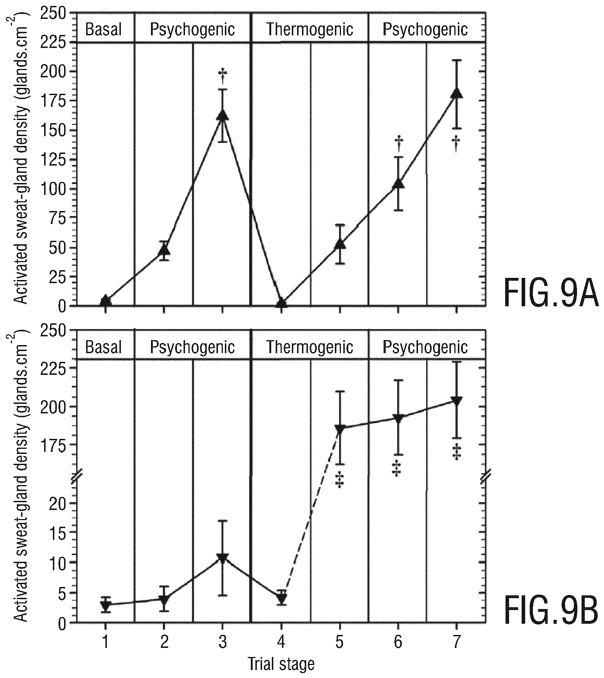

In C.A. Machado-Moreira, N.A.S. Taylor, Thermogenic and psychogenic recruitment of human eccrine sweat glands: Variations between glabrous and non-glabrous skin surfaces, Journal of Thermal Biology 65 (2017), p. 145-152, the thermogenic and psychogenic sweat gland activation is determined for hairy (non-glabrous) and non-hairy (glabrous) skin of the hand. The number of sweat glands per square centimeter that is activated when both thermogenic and psychogenic stimuli are present is about 200 for both skin types. To be more precise, the ratio was measured at the volar (palmar) and dorsal (upper) side of the hand. At the wrist the dorsal side is often hairy and the volar side is not or at least significantly less hairy. For the psychogenic stimulus the number of activated sweat glands per square centimeter is about the same for both thermal conditions: 80 at the volar side and 8 at the dorsal side. For the thermogenic stimulus the number of activated sweat glands per square centimeter is much smaller at the volar side (50) compared to the dorsal side (180). Each activated sweat gland lowers the resistance of the conductance path and hence increases conductance. This is illustrated in Fig. 9A showing a diagram of psychogenic and thermogenic volar activation of sweat glands and Fig. 9B showing a diagram of psychogenic and thermogenic dorsal activation of sweat glands, both averaged over 7 to 10 participants.

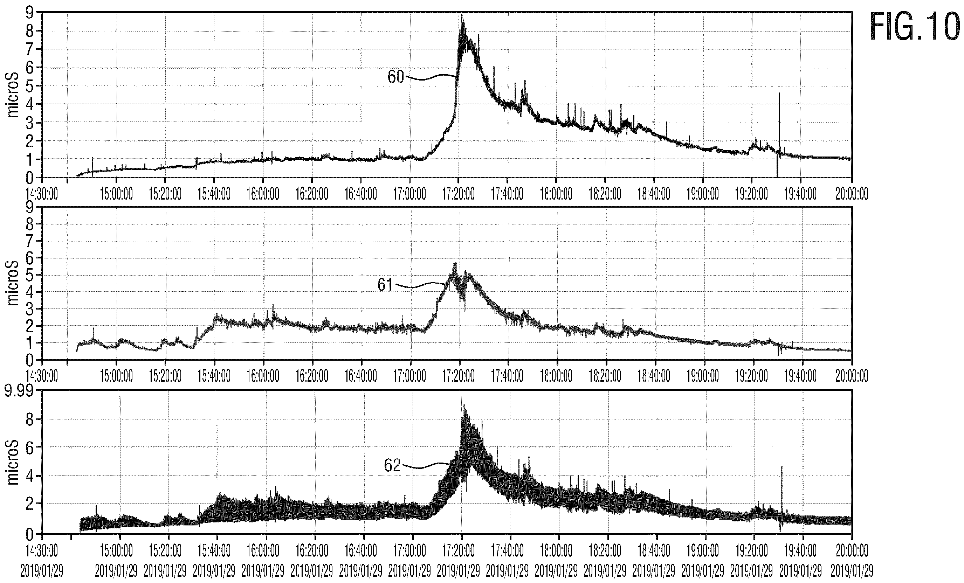

Taking the information described above into account different skin conductances can be expected at the hairy dorsal side of the wrist and the much less hairy volar side. Using the wearable sensor device according to the present invention under similar conditions as in the measurement of Fig. 2 there are indeed differences observed as shown in Fig. 10 that depicts diagrams of skin conductance signals 60-62 measured at the dorsal side of the wrist (signal 60) and at the volar side of the wrist (61) and of a composite of the two skin conductance signals (signal 62). The skin conductances were measured with the device configured to switch the electrode polarity of the three electrodes 11-13 at 80Hz between two configurations: For the measurement signal 60 the two dorsal electrodes 11, 12 have opposite polarity and for the measurement signal 61 the two dorsal electrodes 11, 12 have equal polarity. The measurement signal 62 is a composite of the measurement signals 60 and 61 visualizing the differences between them. The composite may hereby e.g. be signal 60 - c*signal 61. The composite shows all data in one graph, zig zagging between the switching states. The two switching states shown in Fig. 10 correspond to the switching states B and F listed in Table 1.

The skin conductance increases that can be attributed to psychogenic sweat gland responses can be expected, in an exemplary implementation, to be up to approximately 10 times larger at the volar side of the wrist compared to the dorsal side. A ratio of approximately 10 is considered to be the maximum attainable.

The skin conductance increases that can be attributed to thermogenic sweat gland responses can be expected, in an exemplary implementation, to be up to approximately 3.6 times smaller at the volar side of the wrist compared to the dorsal side. The ratio of approximately 3.6 is considered to be the maximum attainable. It shall be noted that this ratio is an approximation based on an measurements on a hand, not a wrist. Similar differences can be seen for measurements at the wrist, but there may be differences between persons and the ratio generally tends to be smaller.

Taking these ratios as a pointer it becomes possible to separate (or deconvolute) the thermogenic and psychogenic contributions to the skin conductance, represented by the inverses of the resistances R1, R2 and R3 (as shown in Figs. 5 and 6).

In one implementation, once the rising edge parameters are known for the switching state(s) having shorted non-glabrous electrodes and the switching state(s) having different polarity non-glabrous electrodes, the simultaneous rising edges are compared with respect to their height (in microSiemens, so prior to normalization). When simultaneous rising edge heights are higher for the traces with switching states of different polarity of the non-glabrous electrodes (upper side of wrist) they can be attributed to thermal stress. The determination of the thermal stress level may be done using solely these rising edges. For the reverse the assignment of the rising edges is to psychogenic stress. After the separation of the rising edges into the two stress types further processing such as normalization may be performed.

In other words, the thermogenic stress response and/or psychogenic stress responses may be determined by evaluating heights of rising edges in the measured skin conductance signals, as described below in more detail. It is considered that an increase in skin conductance results from thermogenic stress if heights of rising edges are higher in a skin conductance signal obtained from a non-glabrous skin region than in a skin conductance signal obtained from a glabrous skin region. It is considered that an increase in skin conductance results from psychogenic stress if heights of rising edges are higher in a skin conductance signal obtained from a glabrous skin region than in a skin conductance signal obtained from a non-glabrous skin region.

The determination of the thermal stress level may thus be done using solely these rising edges. The thermal stress level may be calculated by identifying skin conductance peaks and determining a psychogenic stress level of the user based on normalized parameters of the identified skin conductance peaks. The emotional stress level can be determined similar as the thermal stress level.

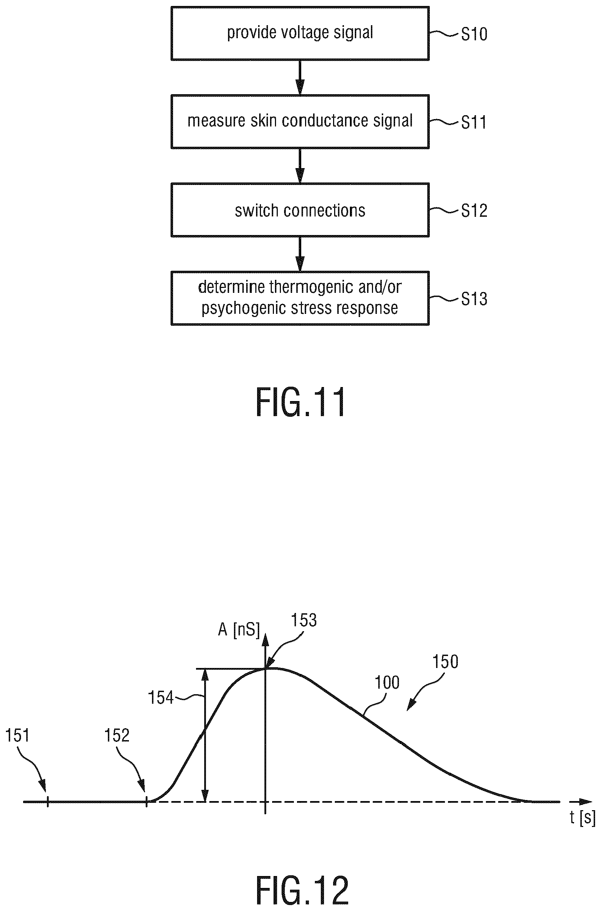

Fig. 11 shows a flow chart of an embodiment of a method according to the present invention. In a first step S10 a voltage signal is provided between a positive input terminal and a negative input terminal of a skin conductance sensor. In a second step S11 a skin conductance signal is measured at an output terminal of the skin conductance sensor. In a third step S12 connections between the first to third electrodes and the positive and negative input terminals of the skin conductance sensor are switched between at least three switching states as explained above. In a fourth step S13 a thermogenic and/or psychogenic stress response is determined from the measured skin conductance signals during the at least three switching states, i.e. the steps S10-S12 are performed at least three times (with different switching states) before step S13 is carried out.

Fig. 12 shows an individual skin conductance response or skin conductance peak 150. The curve in Fig. 12 can be seen as an (extremely) magnified portion of the skin conductance signal trace 100 in Fig. 2. The horizontal axis again denotes the time t, whereas the vertical axis denotes the skin conductance in [nS]. The graph in Fig. 12 spans about 15 seconds, and the graph in Fig. 2 spans about 8 hours. As used herein, a skin conductance peak 150 does not only refer to the maximum point but rather refers to a portion of the respective skin conductance response signal 100. A psychogenic stimulus may occur at the moment denoted by 151. In response to said stimulus, with a slight delay, the skin conductance starts to increase at the onset denoted by 152 until the skin conductance peak 150 reaches its maximum value at 153. The delay can for example be about 1 second for the wrist and about 2 seconds for the ankle. This can be attributed to the signal velocity along the sympathetic nerve. The difference 154 between the skin conductance level at the onset 152 and the skin conductance level at the maximum 153 or peak provides the skin conductance peak or skin conductance response amplitudes ($SCR_{amplitude}$).

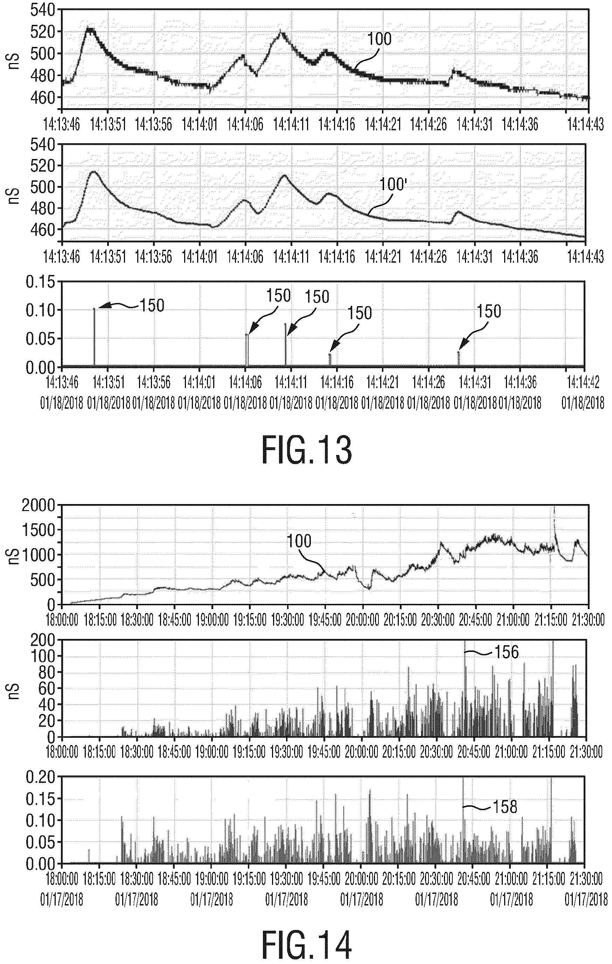

Fig. 13 illustrates signals during different stages of the signal processing. The horizontal axis in the graphs denotes the time, whereas the vertical axis denotes an amplitude. As shown in the upper graph in Fig. 13, the skin conductance signal trace 100 may be measured by the device at a sampling frequency between 40 and 160 Hz. As shown in the middle graph in Fig. 13, this raw signal can optionally be low-pass filtered to yield a smooth down-sampled signal 100' having a sampling rate of, for example, 10 Hz. Subsequently, a plurality of skin conductance peaks 150 can be identified in the skin conductance signal trace. For example, rising edges in the filtered signal can be detected by zero-crossings of the first derivative of the (optionally filtered or preprocessed) skin conductance signal 100.

As an optional further processing step, additional processing and filtering steps can be applied. For example short glitches may be eliminated. In the given example, only rising edges that have a duration longer than 0.8 seconds and shorter than 3.0 seconds are attributed to skin conductance responses (SCR).Further, it is preferred to count every rising edge, even those lasting longer than 3 seconds or only rising edges lasting longer than a predetermined value, for example longer than 0.8 seconds. It has been found that when there are strong emotions, the skin conductance level may rise so fast that no or only too few zero crossings of the first order derivative are observed (e.g., just ripples in the rising edge). Not taking this into account may lead to missing strong emotions. Optionally, the processing device can thus be configured to determine strong emotional responses based on ripples in the rising edge of (a first derivative of) a skin conductance signal trace. It should be noted that skin conductance responses or skin conductance peaks can be identified using known techniques, as for example described in the aforementioned standard textbooks. In the context of the present invention, the expression "stress response" is used for the general processing and the expressions "thermogenic stress responses" or "psychogenic stress responses" are used when the distinction is made between them.

However, in contrast to the generally accepted procedure, the inventors have recognized that by determining, for each of said skin conductance peaks, a normalized parameter of said skin conductance peak, normalized based on a skin conductance value of the respective skin conductance peak, the impact of thermogenic sweating can be reduced, thereby providing a more accurate quantification of psychogenic sweating which may be used to get accurate cortisol responses. For example, a normalized relative skin conductance peak amplitude, also referred to as normalized skin conductance response amplitude can be determined by:

$$SCR_{normalized\_amplitude} = \frac{peak\_level\_value - onset\_level\_value}{onset\_level\_value},$$

wherein the peak (level) value is indicative of a skin conductance level at a peak (see 153 in Fig. 12) of said skin conductance peak and wherein the onset (level) value is indicative of a skin conductance level at the onset (see 152 in Fig. 12) of a skin conductance peak.

The result of this calculation is shown in the lower graph in Fig. 13, wherein the normalized skin conductance response amplitudes, as exemplary a normalized parameters of said skin conductance peak, normalized based on a skin conductance value of the respective skin conductance peak, are denoted by reference numeral 150.

The normalized parameter of the skin conductance peak, here $SCR_{normalized\_amplitude}$ can be a dimensionless number that represents the normalized height of the respective skin conductance response. More generally speaking, determining the normalized parameter can comprise scaling a first value, for example the peak level value, of the skin conductance signal trace at the respective skin conductance peak based on a second value, for example the onset level value, of the skin conductance signal trace at the respective skin conductance peak. It should be noted that the proposed approach differs from what is normally used in the practice of extracting meaningful data from a skin conductance trace. In the aforementioned standard textbook Techniques in Psychophysiology the standard method for obtaining the SCRamplitude provides an absolute amplitude, that is determined by

$$SCR_{absolute\_amplitude} = peak\_level\_value - onset\_level\_value$$

wherein the peak (level) value is indicative of a skin conductance level at a peak (see 153 in Fig. 12) of said skin conductance peak and wherein the onset (level) value is indicative of a skin conductance level at an onset (see 152 in Fig. 12) of the skin conductance peak. In other words, in contrast to the solution proposed herein, the standard method for measuring the amplitude of the skin conductance response only takes the skin conductance level at the top and deducts the skin conductance level at the onset, thus yielding a number with the same dimension as the skin conductance (usually micro Siemens). This (conventional) amplitude can be referred to as absolute skin conductance response amplitude, or $SCR_{absolute\_amplitude}$.

In Fig. 14, the top graph shows a skin conductance signal trace 100, acquired with a Philips discreet tension indicator DTI-5 covering a time frame of 3.5 hours. The horizontal axis denotes the time. The vertical axis denotes the skin conductance in nS (nanoSiemens). The level of the skin conductance 100 gradually rises in this time frame. The middle graph in Fig. 14 represents the non-normalized $SCR_{absolute\_amplitude}$ 156 for each of a plurality of skin conductance peaks and the bottom graph represents the normalized $SCR_{normalized\_amplitude}$ 158 for each of a plurality of skin conductance peaks that were extracted from the skin conductance trace 100. The $SCR_{absolute\_amplitude}$ 156 correlates with the average level of the skin conductance, whereas the $SCR_{normalized\_amplitude}$ 158 does not show this correlation. Nonetheless, even though by the proposed peak-to-peak normalization, the information content is reduced, it has been found that the proposed normalization may eliminate or at least reduce thermogenic influences from the extraction of a (psychogenic) stress level from skin conductance data.

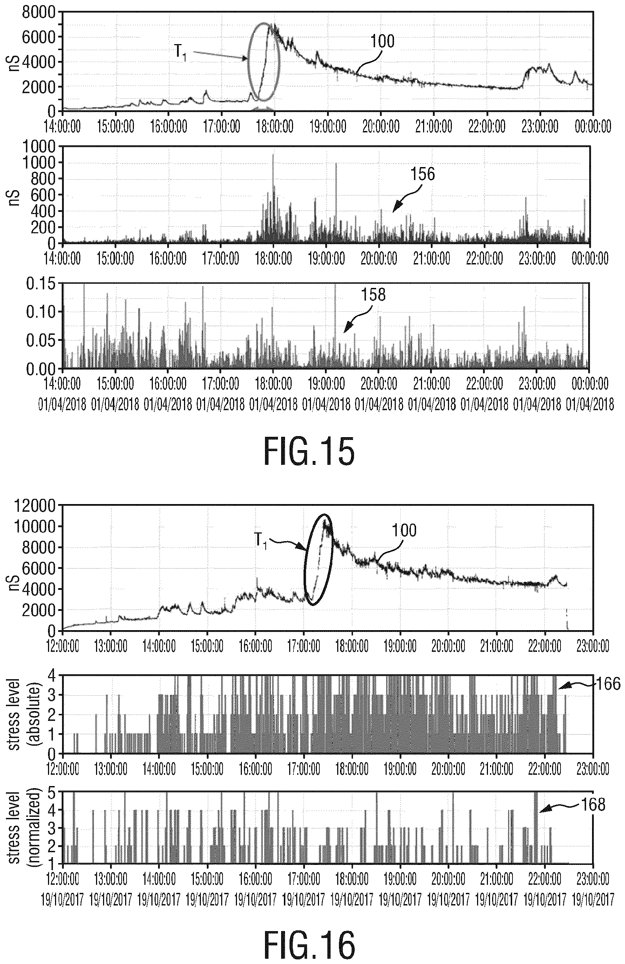

In Fig. 15 this approach is shown for a skin conductance trace 100 that contains thermogenic sweating influences that can be attributed to fast cycling to catch a train in time frame $T_1$, as shown in the upper graph in Fig. 15. The middle graph in Fig. 15 again represents the non-normalized $SCR_{absolute\_amplitude}$ 156 for each of a plurality of skin conductance peaks and the bottom graph represents the normalized $SCR_{normalized\_amplitude}$ 158 for each of a plurality of skin conductance peaks that were extracted from the skin conductance trace 100. It can be seen clearly that the non-normalized $SCR_{absolute\_amplitude}$ 156 increase caused by the cycling activity has negligible influence on the normalized $SCR_{normalized\_amplitude}$ 158 according to the solution proposed herein. Hence, an influence of the skin conductance level variation of the quantification of the skin conductance response amplitude due to thermogenic sweating can be eliminated or at least reduced.

From the middle and bottom graphs in Fig. 15, it also becomes clear that in the two time periods before and after bicycling, the height of the $SCR_{normalized\_amplitude}$ peaks is comparable, truthfully reflecting that their levels of stress are comparable as well. Thus the $SCR_{normalized\_amplitude}$ measure may ensure that psychogenic peaks are given equal weight irrespective of the underlying skin conductance level, which might have greatly increased due to thermogenic sweating, especially at the outside of the wrist.

The conversion of the normalized skin conductance amplitude values to a stress level can be performed using known techniques. For example, a sum of (normalized) rising edge amplitudes per predetermined time interval can be evaluated. Based on a histogram of said sums of (normalized) rising edge amplitudes, different stress levels can be determined and thus user classified or categorized to a corresponding stress level. In the given non-limiting example, five different stress levels are provided, as shown in Fig. 16.

In Fig. 16, the top graph shows a portion of the skin conductance signal trace 100 of the top graph in Fig. 15. The graph again includes a time frame $T_1$ of physical activity, here fast cycling to catch a train. The middle graph in Fig. 16 shows a stress level 166 of the user determined based on a sum of conventional non-normalized rising edge amplitudes (cf. Fig. 15, middle graph). On the other hand, the bottom graph in Fig. 8 shows a stress level 168 of the user determined based on as sum of normalized rising edge amplitudes (cf. Fig. 15, bottom graph). As can be seen from a comparison of the middle and bottom graphs in Fig. 16, the impact of thermogenic sweating that is present during the physical activity from 17:20h onwards. It should be noted that a reduction of mental stress when cycling to the train station also accurately reflects a perceived mental stress level of the user during the measurement.

Referring again to Fig. 15, the inventors have recognized that, further to evaluating the normalized parameters of said skin conductance peaks, as represented in the bottom graph of Fig. 15, the processing unit can also be configured to evaluate a difference between the non-normalized parameters and the normalized parameters. For example, a difference between the non-normalized $SCR_{absolute\_amplitude}$ 156 for each of a plurality of skin conductance peaks and the normalized $SCR_{normalized\_amplitude}$ 158 for each of a plurality of skin conductance peaks that were extracted from the skin conductance trace 100 can be evaluated. Since the non-normalized $SCR_{absolute\_amplitude}$ 156 comprises contributions due to thermogenic sweating and psychogenic sweating and the normalized $SCR_{normalized\_amplitude}$ 158, as a first order approximation, reflects contributions due to psychogenic sweating (only), the difference between the two may yield the effects of thermogenic sweating (only). This can itself be valuable information, for instance, in a top sport where the coach wants to know the level of psychogenic stress of an athlete, which uses up energy otherwise available for the athletic performance.

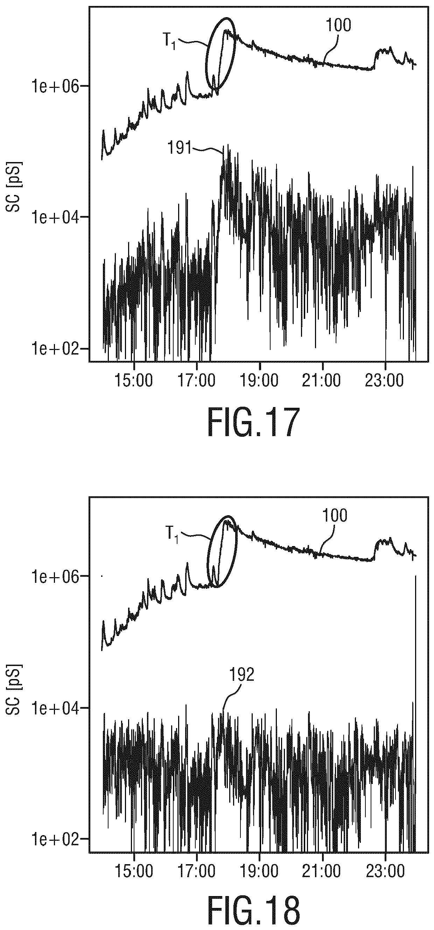

Figs. 17 and 18 show graphs regarding a further embodiment of processing skin conductance data for determining a normalized parameter. Fig. 17 shows a diagram of a skin conductance trace signal (SC) 100 (in [pS] or picoSiemens) and a positive first order derivative or steepness curve 191 of the absolute rising edge heights (in [pS/s]). In Fig. 17, the curve 191 denotes the steepness of the absolute rising edge heights (in [ps/s]) that can be calculated by

$$steepness_{absolute} = SC_{i+1} - SC_i$$

wherein $SC_i$ is the sample value at point i, and $SC_{i+1}$ is the sample value at point i+1. On logarithmic scale, this can be rewritten as:

$$steepness_{absolute,\log} = \left(10\log\left(SC_{i+1} - SC_i\right)\right)\cdot f$$

The curve 191 shown in Fig. 17 shows a moving average over the last 30 samples. In the given example, zero and negative steepness values have been discarded. Nonetheless, as can be seen from Fig. 17, the time interval $T_1$ of physical arousal (here running or cycling to catch a train) shows a strong contribution to the curve 191. Optionally, a maximum rising edge slope of each of a plurality of skin conductance peaks can be identified. Thereby, by only looking at the maximum of the rising edge slope per peak, the amount of data to be stored may be reduced. The terms steepness and slope may be used interchangeably.

Fig. 18 shows a diagram of a skin conductance trace signal 100 (in [pS] or picoSiemens) and a positive first order derivative of a logarithmic transformed skin conductance 192 (in [log10(pS)/s]), as will be described below. It has been found that the thermogenic contribution to the skin conductance data 100 can also be removed by using a measure for the steepness of the rising edges. The steepness can be calculated by taking the difference of logarithmic values of skin conductance measurement i and measurement i+1. Optionally, the value can be multiplied with the sampling:

$$steepness_{normalized,\log} = \left(10\log\left(SC_i\right) - 10\log\left(SC_{i+1}\right)\right)\cdot f$$

wherein $SC_i$ denotes a sample value of the skin conductance signal trace at sample i; $SC_{i+1}$ denotes a sample value of the skin conductance signal trace at a subsequent sample i+1; and f denotes the sampling frequency. The sampling frequency is optional in the aforementioned formulae. Hence, a (positive) first order derivative of the skin conductance (SC) signal 100 after conversion to a logarithmic scale, e.g. by 10log(SC), can be used as an estimator for arousal. It has been found that this estimation may be less sensitive for one or more of the following effects: thermogenic heating, building up micro-climate after mounting, or intense manual labor or exercise. For such a log-calculation, all negative values can be set to zero, leaving only the rising edges of the skin conductance trace. If the logarithm is not used the thermogenic effects are clearly visible in the steepness curve 191 as is shown in Fig. 17. By calculating steepness according to the formula shown above (i.e. by taking a difference of the logarithms) the thermogenic effects are no longer visible in the steepness curve 192 as is shown in Fig. 18. Instead of steepness, it can also be called the first time derivative. Optionally, a normalized maximum rising edge slope of each of a plurality of respective skin conductance peaks can be identified and used for further processing.

It should be noted that the proposed calculation may again be considered as obtaining a normalized parameter of said skin conductance peak, normalized based on a skin conductance value of the respective skin conductance peak. The aforementioned equation may also be rewritten as:

$$steepness = \left(10\log\left(\frac{SC_i}{SC_{i+1}}\right)\right)\cdot f$$

It will be understood that in the aforementioned equations, the sample $SC_{i+1}$ may be replaced by SCi-i and vice versa.

In summary, the present invention provides a stress response measurement at non-glabrous (hairy) skin locations and at a glabrous skin location, at which the impact of thermogenic sweating is less. The present invention may be applied for climate control in vehicles or rooms based on the measurement of thermoregulation and may be used as a clothing advice based on the measurement of thermoregulation.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Wearable sensor device for a thermogenic and/or psychogenic stress response of a subject, the wearable sensor device comprising:

   - a first electrode (11) configured to contact a first non-glabrous skin part of the subject,
   - a second electrode (12) configured to contact a second non-glabrous skin part of the subject,
   - a third electrode (13) configured to contact a glabrous skin part of the subject,
   - a skin conductance sensor (14) configured to provide a voltage signal between a positive input terminal (141) and a negative input terminal (142) and to measure a skin conductance signal at an output terminal (143),
   - a switching arrangement (15) configured to switch connections between the first to third electrodes and the positive and negative input terminals of the skin conductance sensor between at least three switching states, wherein in each switching state two of the first to third electrodes are connected to the negative input terminal and the third of the first to third electrodes is connected to the positive input terminal or in each switching state two of the first to third electrodes are connected to the positive input terminal and the third of the first to third electrodes is connected to the negative input terminal, and
   - a processing unit (15) configured to determine a thermogenic and/or psychogenic stress response from the measured skin conductance signals during the at least three switching states.

2. Wearable sensor device as claimed in claim 1, wherein the switching arrangement (15) is configured to switch connections between the first to third electrodes and the positive and negative input terminals of the skin conductance sensor between six switching states, wherein in each of three switching states two of the first to third electrodes are connected to the negative input terminal and the third of the first to third electrodes is connected to the positive input terminal and in each the other three switching states two of the first to third electrodes are connected to the positive input terminal and the third of the first to third electrodes is connected to the negative input terminal.

3. Wearable sensor device as claimed in claim 1 or 2, wherein the switching arrangement (15) is configured to switch connections between the first to third electrodes and the positive and negative input terminals of the skin conductance sensor, after the at least three switching states, into a DC-free switching state, in which the first to third electrodes are all connected to the negative input terminal or the positive input terminal.

4. Wearable sensor device as claimed in any one of the preceding claims, wherein the switching arrangement (30) comprises three switches (31, 32, 33), each having a first terminal (31a, 32as, 33a) connected to a respective one of the first to third electrodes and two second terminals (31b, 32b, 33b) connected to the positive input terminal and the negative input terminal of the skin conductance sensor.

5. Wearable sensor device as claimed in any one of claims 1 to 3, wherein the switching arrangement (50) comprises six switches (51-56), wherein three switches (51, 52, 53) each have a first terminal (51a, 52a, 53a) connected to a respective one of the first to third electrodes and a second terminal (51b, 52b, 53b) connected to the positive input terminal of the skin conductance sensor and the other three switches (54, 55, 56) each have a first terminal (54a, 55a, 56a) connected to a respective one of the first to third electrodes and a second terminal (54b, 55b, 56b) connected to the negative input terminal of the skin conductance sensor.

6. Wearable sensor device as claimed in any one of the preceding claims, wherein the processing unit (16) is configured to determine the thermogenic stress response and/or psychogenic stress responses by evaluating heights of rising edges in the measured skin conductance signals.

7. Wearable sensor device as claimed in any one of the preceding claims, wherein the processing unit (16) is configured to determine that an increase in skin conductance results from thermogenic stress if heights of rising edges are higher in a skin conductance signal obtained from a non-glabrous skin region than in a skin conductance signal obtained from a glabrous skin region and/or to determine that an increase in skin conductance results from psychogenic stress if heights of rising edges are higher in a skin conductance signal obtained from a glabrous skin region than in a skin conductance signal obtained from a non-glabrous skin region.

8. Wearable sensor device as claimed in any one of the preceding claims,
wherein the switching arrangement (15) is configured to switch the connections at a switching rate that is an integer fraction of the sampling rate of the measured skin conductance signal.

9. Wearable sensor device as claimed in any one of the preceding claims,
wherein the first, second and third electrodes (21, 22, 23) are integrated into a single device.

10. Wearable sensor device as claimed in any one of claims 1 to 8,
wherein the first, second and third electrodes (11, 12, 13) are configured to be separately arranged at the subject's body.

11. Wearable sensor device as claimed in claim 10,
wherein the processing unit (16) is configured to synchronize evaluation of the skin conductance signals measured in the different switching states.

12. Wearable sensor device as claimed in any one of the preceding claims,
further comprising a strap (25) configured to be wound around the subject's wrist and a housing (24) held by the strap and housing the switching arrangement, the skin conductance sensor and the processing unit,
wherein the first and second electrodes (21, 22) are arranged in or at a bottom surface of the housing facing the dorsal side of the wrist when the wearable sensor device is worn by the subject, and
wherein the third electrode (23) is arranged in or at a position of the strap facing the volar side of the wrist when the wearable sensor device is worn by the subject.

13. Wearable sensor device as claimed in any one of the preceding claims,
wherein the switching arrangement (30) is configured to apply switching states, which individually or on average have an equal duration.

14. Sensing method of a wearable sensor device for determining a thermogenic and/or psychogenic stress response measuring skin conductance of a subject, the wearable sensor device comprising a first electrode (11) configured to contact a first non-glabrous skin part of the subject, a second electrode (12) configured to contact a second non-glabrous skin part of the subject, and a third electrode (13) configured to contact a glabrous skin part of the subject, the sensing method comprising:

- providing a voltage signal between a positive input terminal (141) and a negative input terminal (142) of a skin conductance sensor (14),
- measuring a skin conductance signal at an output terminal (143) of the skin conductance sensor (14),
- switching connections between the first to third electrodes and the positive and negative input terminals of the skin conductance sensor between at least three switching states, wherein in each switching state two of the first to third electrodes are connected to the negative input terminal and the third of the first to third electrodes is connected to the positive input terminal or in each switching state two of the first to third electrodes are connected to the positive input terminal and the third of the first to third electrodes is connected to the negative input terminal, and
- determining a thermogenic and/or psychogenic stress response from the measured skin conductance signals during the at least three switching states.

15. Computer program comprising program code means for causing a computer to carry out the steps of the method as claimed in claim 14 when said computer program is carried out on the computer.

FIG.1

FIG.2

FIG.3

FIG.4

FIG.5

Dorsal side of wrist

FIG.6

Volar side of wrist

FIG.7

FIG.8

FIG.9A

FIG.9B

FIG.10

EP 3 834 716 A1

| provide voltage signal | ~S10 |

↓

| measure skin conductance signal | ~S11 |

↓

| switch connections | ~S12 |

↓

| determine thermogenic and/or psychogenic stress response | ~S13 |

# FIG.11

# FIG.12

FIG.13

FIG.14

FIG.15

FIG.16

FIG.17

FIG.18

Europäisches
Patentamt

European
Patent Office

Office européen
des brevets

# EUROPEAN SEARCH REPORT

Application Number

EP 19 21 5419

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2019/073756 A1 (SONY CORP [JP]) 18 April 2019 (2019-04-18) * abstract * * paragraph [0022] - paragraph [0045] * * figures 1-37 * | 1-15 | INV. A61B5/053 A61B5/16 A61B5/00 |
| A | US 2019/282152 A1 (OUWERKERK MARTIN [NL]) 19 September 2019 (2019-09-19) * the whole document * | 1-15 | |
| A | KR 101 828 068 B1 (YUJEONG SYSTEM [KR]) 12 February 2018 (2018-02-12) * the whole document * | 1-15 | |
| A | US 2019/320981 A1 (KODAMA KEI [JP] ET AL) 24 October 2019 (2019-10-24) * paragraph [0010] - paragraph [0018] * * paragraph [0045] - paragraph [0098] * * figures 1-23 * | 1-15 | |

TECHNICAL FIELDS
SEARCHED (IPC)

A61B

The present search report has been drawn up for all claims

| Place of search | Date of completion of the search | Examiner |
|---|---|---|
| Berlin | 14 May 2020 | Abraham, Volkhard |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 21 5419

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2020

| Patent document cited in search report | | Publication date | Patent family member(s) | | Publication date |
|---|---|---|---|---|---|
| WO 2019073756 | A1 | 18-04-2019 | NONE | | |
| US 2019282152 | A1 | 19-09-2019 | CN | 109640811 A | 16-04-2019 |
| | | | EP | 3484360 A1 | 22-05-2019 |
| | | | JP | 2019527097 A | 26-09-2019 |
| | | | US | 2019282152 A1 | 19-09-2019 |
| | | | WO | 2018015308 A1 | 25-01-2018 |
| KR 101828068 | B1 | 12-02-2018 | NONE | | |
| US 2019320981 | A1 | 24-10-2019 | JP | WO2018105447 A1 | 24-10-2019 |
| | | | US | 2019320981 A1 | 24-10-2019 |
| | | | WO | 2018105447 A1 | 14-06-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 10085695 B

**Non-patent literature cited in the description**

• **M. OUWERKERK ; P. DANDINE ; D. BOLIO ; R. KOCIELNIK ; J. MERCURIO ; H. HUIJGEN ; J. WESTERINK.** Wireless multi sensor bracelet with discreet feedback. *Proceedings of Wireless Health,* 2013

• **C.A. MACHADO-MOREIRA ; N.A.S. TAYLOR.** Thermogenic and psychogenic recruitment of human eccrine sweat glands: Variations between glabrous and non-glabrous skin surfaces. *Journal of Thermal Biology,* 2017, vol. 65, 145-152